# EUROPEAN PATENT APPLICATION

(11) **EP 3 502 121 A1**
(43) Date of publication of application: **26.06.2019**
(21) Application number: 16913262.8
(22) Date of filing: 19.08.2016
(51) Int. Cl.: C07K 1/18, C07K 1/36, C07K 7/62, A61K 38/12, A61P 31/04

(54) **POLYMYXIN B SULPHATE CRYSTAL AND PREPARATION METHOD THEREFOR**

(71) Applicant: Hubei Ruihaoanke Pharmaceutical Technology Development Co., Ltd., Wuhan, Hubei 430071 (CN)
(72) Inventor: WANG, Zengxia, Wuhan Hubei 430071 (CN); LI, Xiaobing, Wuhan Hubei 430071 (CN); LUO, Fen, Wuhan Hubei 430071 (CN); LI, Changhong, Wuhan Hubei 430071 (CN); XU, Yanwei, Wuhan Hubei 430071 (CN); CHEN, Long, Wuhan Hubei 430071 (CN)
(74) Representative: Uexküll & Stolberg
(86) International application number: PCT/CN2016/096063
(87) International publication number: WO 2018/032506

(57) **Abstract**

The present invention provides a polymyxin B sulfate crystal and a preparation method thereof. Said method comprises the following steps of: adsorbing a polymyxin B fermentation liquid with a resin, and adding sulfuric acid or acidic ethanol for desorbing; concentrating to obtain a saturated solution of polymyxin B sulfate; and using an organic solvent to precipitate a crystal from the saturated solution, filtering and drying to obtain the polymyxin B sulfate crystal.

## Description

### Technical field

The present invention belongs to the technical field of pharmacy, and in particular relates to a polymyxin B sulfate crystal and a preparation method thereof.

### Background Art

Polymyxin B is an alkaline cyclic polypeptide antibiotic produced by *bacillus polymyxa* and composed of various amino acids and fatty acids. Polymyxin B product is a multi-component mixture, including polymyxin B1, B2, B3, B1-I (it is required that the sum of the mass of these four components ≥80.0% in the European Pharmacopoeia), and its sulfate is commonly used clinically. Polymyxin B sulfate is a white or off-white powder with hygroscopicity. Polymyxin B sulfate has a strong killing effect on Gram-negative bacteria, and in particular, it renders high *in vitro* sensitivity to NDM-1 bacteria (super bacteria), and thus has attracted much attention.

As for the refining of polymyxin B sulfate product, it is usually carried out by a spray drying method (patent application CN201210519331.7) or a lyophilization method (patent application CN201510775580.6) due to its difficulty to crystallize. The current patent about polymyxin B crystal includes polymyxin B1 dihydrate crystal disclosed in the patent application CN201210379231.9, which is obtained by precipitation using a mixed solvent of acetone and diethyl ether. But diethyl ether is extremely volatile and easily to be oxidized in air and causes an explosion, which is not suitable for industrial production. In addition, the products on the market are all mixtures of polymyxin B sulfate. Regarding the preparation of polymyxin B1 sulfate monomer, the patent application filed by the present applicant has been granted (Patent No. ZL201110390624.5), and the final product is also prepared by spray drying method. Both the polymyxin B sulfate prepared by spray drying method and the commercially available polymyxin B sulfate product have strong hygroscopicity, the solution of which is tested to exhibit a low clarity and certain turbidity, and the product quality needs to be improved. Crystallization of the mother liquor by conventional crystallization method can remove some impurities, but the precipitate of polymyxin B sulfate is very sticky and easy to agglomerate, making it difficult to carry out the crystallization operation. Therefore, it is necessary to develop a new polymyxin B sulfate crystal and a preparation method thereof.

### Description of the Invention

In view of the problems that the polymyxin B sulfate product in the prior art is not high in clarity and purity, and the crystal precipitate is easily to agglomerate, the present invention provides a polymyxin B sulfate crystal and a preparation method thereof.

The method for preparing a polymyxin B sulfate crystal provided by the present invention comprises the following steps of:
1) adsorbing a polymyxin B fermentation liquid with a resin, and adding a sulfuric acid aqueous solution and/or an acidic ethanol aqueous solution for desorbing to obtain a desorption liquid;
2) adjusting the pH of the desorption liquid obtained from step 1) to 5.0-7.0, and then concentrating until a solid precipitates to obtain a saturated solution of polymyxin B sulfate;
3) adding an organic solvent dropwise into the saturated solution of polymyxin B sulfate obtained from step 2), or adding the saturated solution of polymyxin B sulfate obtained from step 2) dropwise into an organic solvent while stirring to precipitate a crystal, filtering, and drying the filter cake to obtain a polymyxin B sulfate crystal.

Preferably, in step 1) of the method of the present invention, the concentration of said sulfuric acid aqueous solution is 0.2 mol/L.

According to a specific embodiment of the method of the present invention, in step 1), the desorbing process is desorbing at a rate of 0.5 column volume of 0.2 mol/L sulfuric acid aqueous solution per hour, and the desorption liquid is fractionally collected. The collection is started when the desorption solution potency is higher than 500 µg/ml, and the collection is stopped when the potency is less than 500 µg/ml.

Preferably, in step 1) of the method of the present invention, before adsorbing a polymyxin B fermentation liquid with a resin, said polymyxin B fermentation liquid is subjected to a pretreatment comprising the following steps of:
a) acidifying;
b) adding celite and filtering.

Preferably, in step 1) of the method of the present invention, said adsorbing a polymyxin B fermentation liquid with a resin comprises:
(i) ion exchange treatment;
(ii) macroporous adsorption resin treatment.

According to a specific embodiment of the method of the present invention, said pretreatment comprises adjusting the pH of the polymyxin B fermentation liquid to 1.8, then adding celite, filtering after stirring homogeneously, and top washing with water until the filtrate potency is less than 0.2 g/L; preferably, said acidifying is adjusting the pH of the polymyxin B fermentation liquid to 1.8 by adding oxalic acid, then adding celite, frame filtering after stirring homogeneously, and top washing with water until the filtrate potency is less than 0.2 g/L.

According to a specific embodiment of the method of the present invention, said ion exchange treatment comprises adjusting the pH of the filtrate of the polymyxin B fermentation liquid treated by acidifying to 6.7, and then adsorbing with a weakly acidic ion exchange resin, and after completely adsorbing, washing with 2-3 volumes of water in terms of column volume (i.e. the volume of the weakly acidic ion exchange resin in the column) until the effluent is colorless, and then desorbing with a sulfuric acid aqueous solution. Preferably, said weakly acidic ion exchange resin is LXD-135 weakly acidic ion exchange resin. More specifically, said ion exchange treatment comprises adjusting the pH of the filtrate of the polymyxin B fermentation liquid pretreated by acidifying to 6.7 with 2M sodium hydroxide solution, then adsorbing with a weakly acidic ion exchange resin, and after completely adsorbing, washing with 2-3 column volumes of water until the effluent is colorless, and then desorbing with0.2M sulfuric acid aqueous solution; and preferably, said weakly acidic ion exchange resin is LXD-135 weakly acidic ion exchange resin.

According to a specific embodiment of the method of the present invention, said macroporous adsorption resin treatment comprises adjusting the pH of the ion exchange treated polymyxin B fermentation liquid to 6.5, and after completely adsorbing by macroporous adsorption resin, top washing with 2 volumes of water in terms of column volume, and then desorbing with an acidic ethanol aqueous solution; preferably, said macroporous adsorption resin treatment comprises adjusting the pH of the ion exchange treated polymyxin B fermentation liquid to 6.5 with a 2M sodium hydroxide solution, and after completely adsorbing by macroporous adsorption resin, top washing with 2 volumes of water in terms of column volume, and then desorbing with a 40% (v/v) ethanol aqueous solution having a pH of 3.

Preferably, the method of the present invention further comprises in step 1), concentrating the obtained desorption liquid, decolorizing it by activated carbon, and filtering.

Preferably, in step 2) of the method of the present invention, the pH of the desorption liquid is adjusted to 5.0-7.0 with a 0.5M-10M sodium hydroxide solution.

Preferably, in step 3) of the method of the present invention, said organic solvent is selected from one or more of C₁-C₄ alcohol, C₃-C₄ ketone, ethyl acetate or butyl acetate; more preferably, said C₁-C₄ alcohol is selected from one or more of methanol, ethanol, isopropanol, n-propanol or butanol; and more preferably, said C₃-C₄ ketone is selected from acetone or 2-butanone.

Preferably, in step 3) of the method of the present invention, the process of precipitating the crystal is carried out at a temperature of 0-40 °C.

According to a specific embodiment of the method of the present invention, the method for preparing a polymyxin B sulfate crystal comprises the following steps of:
1) acidifying a polymyxin B fermentation liquid, adding celite and filtering, ion exchange treating, macroporous adsorption resin treating, followed by adding a sulfuric acid aqueous solution for desorbing to obtain a desorption liquid; and concentrating the obtained desorption liquid, decolorizing it by activated carbon, and then filtering to obtain a filtrate;
2) adjusting the pH of the filtrate to 5.0-7.0 with a sodium hydroxide aqueous solution, and concentrating until a solid precipitates to obtain a saturated solution of polymyxin B sulfate; and
3) adding an organic solvent dropwise into the saturated solution of polymyxin B sulfate obtained from step 2) while stirring until a crystal precipitates, and holding the temperature during adding dropwisein the range of 0-10 °C; and continuing adding 0.5-5 volumes of the organic solvent in terms of the volume of the saturated solution of polymyxin B sulfate dropwise and stirring for 0-8 hours, filtering, discarding the filtrate, and drying the filter cake under vacuum for 3-20 hours to obtain a crystalline polymyxin B sulfate.

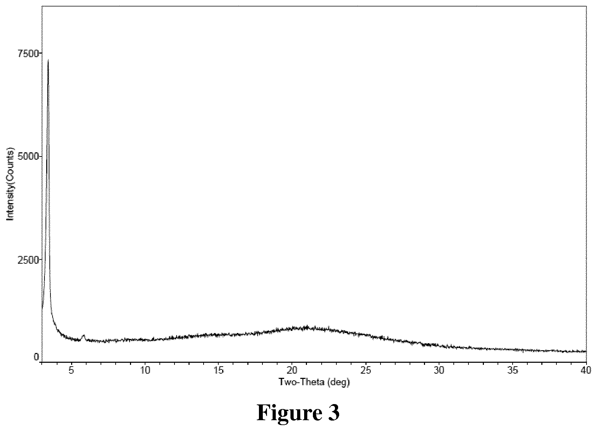

The present invention also provides a crystalline polymyxin B sulfate prepared according to the above method, that is, the present invention also provides a polymyxin B sulfate crystal, wherein the crystal has an X-ray powder diffraction pattern expressed by 2θ degree using Cu-Ka radiation as shown in Figure 3. As can be seen from Figure 3, the polymyxin B sulfate prepared according to the method of the present invention is crystalline.

Further, the present invention also provides a pharmaceutical composition, said pharmaceutical composition comprises the polymyxin B sulfate crystal and a pharmaceutically acceptable carrier. Optionally, the pharmaceutical composition further comprises one or more antibacterial active ingredients other than polymyxin B sulfate. Preferably, said pharmaceutical composition is in the form of tablet, capsule, or granule; more preferably, said tablet is selected from rapid-release tablet, chewable tablet, dispersible tablet, effervescent tablet, sustained release tablet, controlled release tablet or enteric coated tablet, said capsule is selected from hard capsule, soft capsule, sustained release capsule, controlled release capsule or enteric coated capsule, and said granule is selected from suspension granule, effervescent granule, enteric coated granule, sustained release granule or controlled release granule.

The present invention also provides the use of the polymyxin B sulfate crystal in the preparation of an antibacterial drug, preferably in the preparation of a medicament against Gram-negative bacteria. The present invention also provides the use of the polymyxin B sulfate crystal in the preparation of a medicament against drug-resistant bacteria, in particular Gram-negative drug-resistant bacteria, such as Gram-negative super drug-resistant bacteria.

In other words, the present invention also provides a polymyxin B sulfate crystal for use in resisting bacteria, in particular Gram-negative bacteria. The present invention also provides a polymyxin B sulfate crystal for use in resisting drug-resistant bacteria, in particular Gram-negative drug-resistant bacteria, such as Gram-negative super drug-resistant bacteria.

The present invention also provides a method for preventing and/or treating a bacterial infection, in particular a disease or condition caused by Gram-negative bacteria, the method comprises administering to a subject or patient in need thereof a prophylactically and/or therapeutically effective amount of pharmaceutical preparation comprising the polymyxin B sulfate crystal.

The present invention also provides a method for preventing and/or treating drug-resistant bacteria infection, in particular a disease or condition caused by Gram-negative drug-resistant bacteria, such as Gram-negative super drug-resistant bacteria, the method comprises administering to a subject or patient in need thereof a prophylactically and/or therapeutically effective amount of pharmaceutical preparation comprising the polymyxin B sulfate crystal.

The present invention provides a polymyxin B sulfate crystal product, and the experimental results show that the product exhibits a crystal form, has better clarity and purity, and has low hygroscopicity. In addition, the preparation method provided by the present invention is easy to operate, and the solvent used for crystallization is low in cost and easy to obtain, which is suitable for large-scale industrial production, and lays a foundation for extending clinical application of polymyxin B sulfate crystal.

### Brief Description of the Drawings

Figure 1A shows the crystal of polymyxin B sulfate in n-butanol-ethanol aqueous solution;
Figure 1B shows a sample of polymyxin B sulfate crystal after drying;
Figure 2A is a micrograph of polymyxin B sulfate crystal at a magnification of 40X;
Figure 2B is a micrograph of polymyxin B sulfate crystal at a magnification of 100X;
Figure 3 is an X-ray powder diffraction (XRD) pattern of a sample of polymyxin B sulfate crystal;
Figure 4A is an HPLC chromatogram and chromatographic peak data of a spray dried sample of polymyxin B sulfate;
Figure 4B is an HPLC chromatogram and chromatographic peak data of a sample of polymyxin B sulfate crystal;
Figure 5 shows the comparison of clarity of the polymyxin B sulfate crystal (left) with a spray dried sample (right).

### Best Mode for Carrying Out the Invention

The present invention will be further described in conjunction with the accompanying drawings and the embodiments to achieve better illustration of the present invention and to facilitate understanding of the technical solution of the present invention. It is to be understood that the specific examples are only intended to illustrate the present invention and are not intended to limit the scope of the present invention.

In the present application, the polymyxin B fermentation liquid used is prepared by pure strain fermentation for 40-50 hours using *bacillus polymyxa* with flour and cottonseed meal as main raw materials to obtain the polymyxin B fermentation liquid. The control sample is a polymyxin B sulfate raw material sample and a commercially available sample.

### Example 1

Into 5L polymyxin B fermentation liquid with a potency of 1.8 g/L was added oxalic acid solid to adjust the pH to 1.8. After stirring for 30 minutes, 250 g celite was added, and stirring was continued for 30 minutes. Then the resulting mixture was frame filtered and top washed with water to a potency of less than 0.2 g/L. The filtration was stopped to obtain 7.1 L filtrate, which contained 8.5 g polymyxin B. The filtrate was adjusted to pH 6.7 with a 2M sodium hydroxide solution and then loaded onto a chromatography column containing 60 ml LXD-135 weakly acidic ion exchange resin to pass through the column for adsorption. After completely adsorbing, the column was washed with 2-3 volumes of water in terms of the volume of LXD-135 weakly acidic ion exchange resin until the effluent was colorless, and then desorbing was carried out with a 0.2M sulfuric acid aqueous solution to obtain 800ml desorption liquid (containing 7.4 g polymyxin B sulfate). The desorption liquid was adjusted to pH 6.5 with a 2M sodium hydroxide solution, and then loaded onto a column containing 80 ml LXT-081 macroporous adsorption resin for adsorption. After completely adsorbing, the column was top washed with 2 volumes of water in terms of column volume, and then desorbed with a 40% (v/v) acidic ethanol aqueous solution (pH 3) to obtain 1600 ml desorption liquid (containing 6.74 g polymyxin B sulfate).

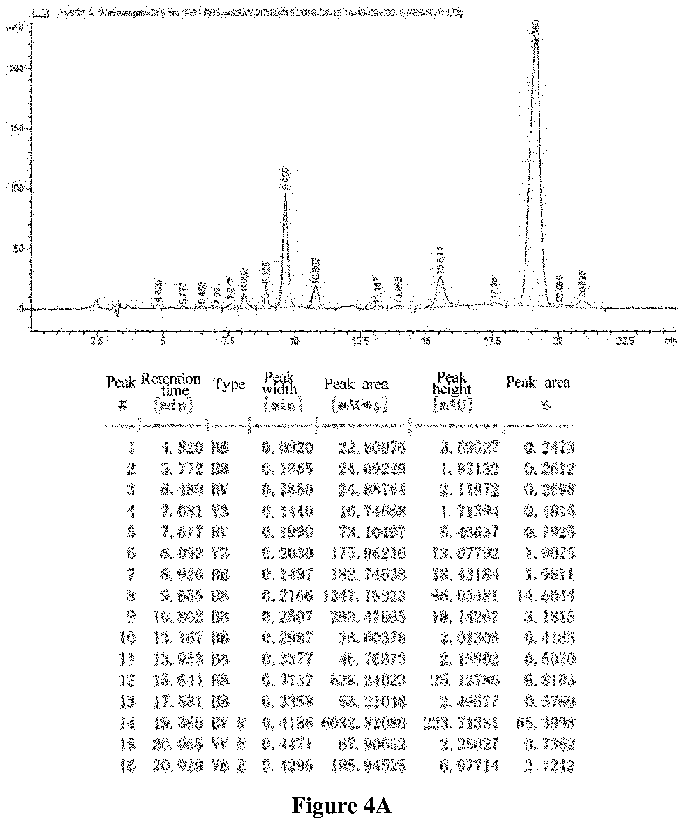

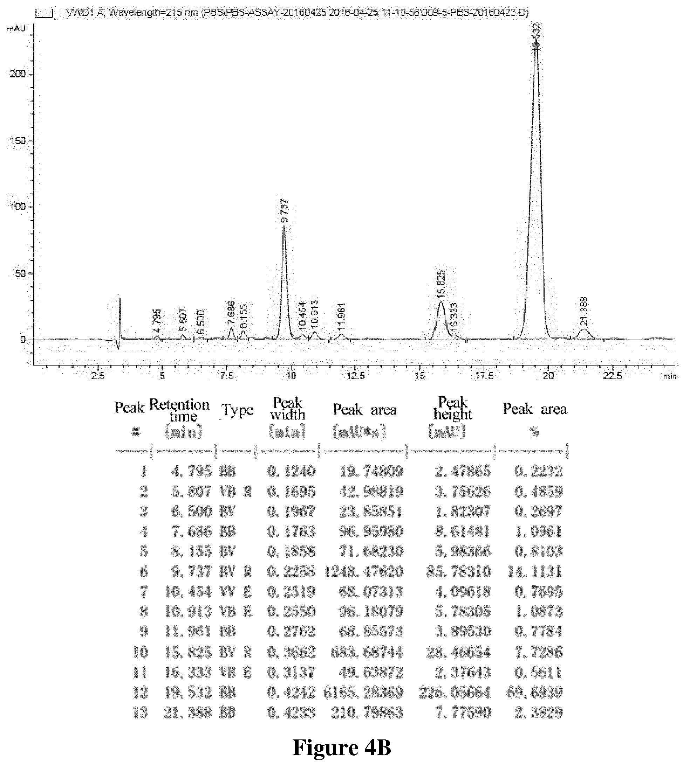

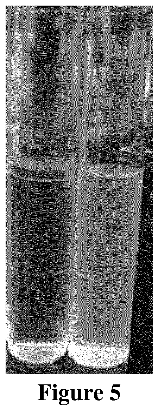

After the resulting desorption liquid was concentrated to 1/3 solution volume by a rotary evaporator at a temperature of 50 °C, 5g 1% (w/v) activated carbon by volume of the concentrate was added, the resulting mixture was stirred at 60 °C for 30 minutes, and filtered. The filtrate contained 6.1 g polymyxin B. The filtrate was adjusted to pH 5.0-7.0 with a 2M sodium hydroxide solution, and then the concentrating is continued to a volume of about 70 ml. Stirring was started, and anhydrous ethanol was slowly added dropwise until the solution was turbid, the dropwise adding of anhydrous ethanol was stopped, and the system was cooled to 0-5 °C. Stirring was continued until the solid precipitated, and 1-2 volumes of anhydrous ethanol in terms of the volume of the concentrate was continued to be added dropwise until a total of 3 volumes of anhydrous ethanol in terms of the volume of the concentrate was added dropwise. Stirring was then continued for 6 hours at 0-5 °C, and the solid was filtered off. The filtered solid was dried under vacuum at a temperature of 60 °C for 4 hours to obtain a white solid (5.4 g), crystal yield: 88.5%, purity: 91.38%. The X-ray powder diffraction (XRD) pattern of the obtained polymyxin B sulfate crystal is shown in Figure 3. The result of clarity test (Chinese Pharmacopoeia (2010 Edition) Volume II, Appendix IXB) is less than No. 1 (as shown on the left panel of Figure 5), while the clarity of commercially available polymyxin B sulfate is greater than No. 2 (as shown on the right panel of Figure 5). The polymyxin B sulfate prepared by the method of the present invention has a potency of 8300 U/g, which is measured by the cup-plate method. The HPLC chromatograms of the obtained polymyxin B sulfate crystal sample and the spray dried sample are shown in Figure 4B and Figure 4A, respectively.

### Example 2

5L Polymyxin B fermentation liquid with a potency of 1.8 g/L was taken, and then a desorption liquid was prepared according to the procedure of Example 1. The desorption liquid obtained in this example was concentrated to a concentrate with a volume of 50 ml (containing 5.3 g polymyxin B sulfate). Stirring was started, and ethyl acetate was slowly added dropwise to the concentrate until the solution was turbid, the dropwise adding of ethyl acetate was stopped, and then the system was cooled to 0-5 °C. Stirring was continued until the solid precipitated, and then the remaining ethyl acetate was continued to be added dropwise until a total of 3 volumes of ethyl acetate in terms of the volume of the concentrate was added dropwise. Stirring was then continued for 7 hours at 0-5 °C, and the solid was filtered off. The filtered solid was dried under vacuum at a temperature of 60 °C for 3 hours to obtain a white solid (4.82 g), crystal yield: 91%, purity: 89.58%. The result of clarity test is No. 1, and the potency measured by the cup-plate method is 8355 U/g.

### Example 3

7L Polymyxin B fermentation liquid with a potency of 1.8 g/L was taken, and then a desorption liquid was prepared according to the procedure of Example 1. The desorption liquid of this example was concentrated to a concentrate with a volume of 85 ml (containing 6.7 g polymyxin B sulfate). Stirring was started, and acetone was slowly added dropwise to the concentrate until the solution was turbid, the adding dropwise of acetone was stopped. Then the system was cooled to 0-5 °C. Stirring was continued until the solid precipitated, and then the remaining acetone was continued to be added dropwise until a total of 3.5 volumes of acetone in terms of the volume of the concentrate was added dropwise. Stirring was then continued for 6 hours at 0-5 °C, and the solid was filtered off. The filtered solid was dried under vacuum at a temperature of 60 °C for 4 hours to obtain a white solid (5.83 g), crystal yield: 87%, purity: 90.98%, clarity: No. 1, and the potency measured by the cup-plate method is 8100 U/g. The crystal sample of the present example and the commercially available product were subjected to biological potency test and HPLC detection under the same condition. The results show that the biological potency of the crystal sample of the present example is 8.8% higher than that of the commercially available product. Since it is difficult to increase the biological potency by 8.8% over commercially available product, the increase in biological potency of the polymyxin B sulfate crystal product of the present invention is great in terms of product quality. The chromatographic purity of main components (B1, B2, B3, B1-I) in the HPLC result of the commercially available product (prepared by spray drying) is 89.99% (Figure 4A), while the chromatographic purity of active ingredients (B1, B2, B3, B1-I) in the HPLC result of the crystal sample of the present invention is 92.62% (Figure 4B), which is much higher than the requirement that the sum of the four components ≥80.0% in the European Pharmacopoeia. Compared with the spray drying process currently commonly used, the method of the present invention can significantly improve the chromatographic purity and content of the crystal product, thereby improving the product quality.

### Example 4

4L Polymyxin B fermentation liquid with a potency of 1.5 g/L was taken, and then a desorption liquid was prepared according to the procedure of Example 1. The desorption liquid of this example was concentrated to a saturated solution with a volume of 25 ml (containing 2.60 g polymyxin B sulfate, measured by sampling). Then, the saturated solution was slowly added dropwise into 250 ml (10 volumes in terms of the volume of the saturated solution of polymyxin B sulfate) isopropanol under stirring. The temperature of isopropanol during adding was always controlled within the range of 5-10 °C. The polymyxin B sulfate crystals precipitated during adding gradually increased, which was in a uniform dispersion state without adhesion. Stirring was continued for 1 hour, the mixture was filtered, and the filtered solid was dried under vacuum at a temperature of 50 °C for 10 hours to obtain a crystal powder of polymyxin B sulfate (2.40 g), crystal yield: 92.3%.

### Example 5

10L Polymyxin B fermentation liquid with a potency of 2.0 g/L was taken, and then a desorption liquid was prepared according to the procedure of Example 1. The desorption liquid of this example was concentrated to a saturated solution with a volume of 140 ml (containing 13.50 g polymyxin B sulfate, measured by sampling). Then, the saturated solution was slowly added dropwise into 700ml (5 volumes in terms of the volume of the saturated solution of polymyxin B sulfate) n-butanol under stirring. The temperature of the n-butanol solution system during adding was always controlled within the range of 25-30 °C. The polymyxin B sulfate crystals precipitated during adding gradually increased, which was uniformly dispersed without adhesion and had a good granularity. After dropwise addition of the aqueous solution of polymyxin B sulfate was completed, stirring was continued for 30 min, the mixture was filtered, and the filtered solid was dried under vacuum at a temperature of 40 °C for 20 hours to obtain a crystal powder of polymyxin B sulfate (12.40 g), crystal yield: 91.9%.

### Example 6

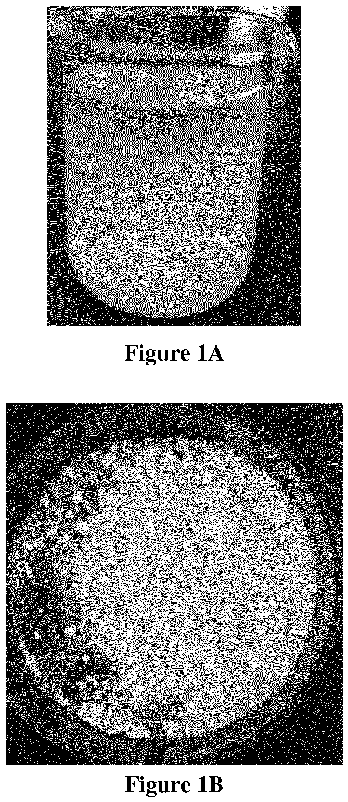

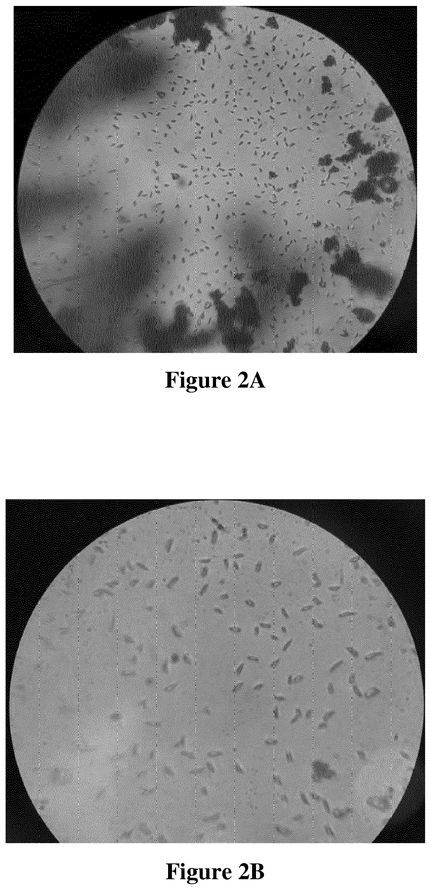

7L Polymyxin B fermentation liquid with a potency of 1.5 g/L was taken, and then a desorption liquid was prepared according to the procedure of Example 1. The desorption liquid was concentrated to a saturated solution with a volume of 50 ml (containing 5.00 g polymyxin B sulfate, measured by sampling). Then, the saturated solution was slowly added dropwise into 400 ml (8 volumes in terms of the volume of the saturated solution of polymyxin B sulfate) mixed solvent of ethanol (200 ml) and n-butanol (200 ml) under stirring. The temperature during adding was always controlled within the range of 0-5 °C. The polymyxin B sulfate crystals precipitated during adding gradually increased, which was uniformly dispersed with a good granularity (Figure 1). After dropwise addition of the aqueous solution of polymyxin B sulfate was completed, stirring was continued for 2 hours, the mixture was filtered, and the filtered solid was dried under vacuum at a temperature of 60 °C for 8 hours to obtain a crystal powder of polymyxin B sulfate (4.52 g), crystal yield: 90.4%. It was observed by microscope at a magnification of 40X and 100X that the polymyxin B sulfate crystals were very obvious, and the individual crystal was uniform (Figure 2A and Figure 2B).

### Example 7

15 L Polymyxin B fermentation liquid with a potency of 1.5 g/L was taken, and then a desorption liquid was prepared according to the procedure of Example 1. The desorption liquid was concentrated to a saturated solution with a volume of 100 ml (containing 10.00 g polymyxin B sulfate, measured by sampling). Then, the saturated solution was slowly added dropwise into 1000 ml (10 volumes in terms of the volume of the saturated solution of polymyxin B sulfate) mixed solvent of ethanol (500 ml) +isopropanol (500ml) under stirring. The temperature during adding was always controlled within the range of 5-10 °C. After the crystals were precipitated, the remaining solvent was continuously added dropwise, to obtain crystals which were uniformly dispersed and had a good granularity, with solid-liquid separation being quickly achieved after standing (30 min). Stirring was continued for 30 min, the mixture was filtered, and the filtered solid was dried under vacuum at a temperature of 55 °C for 10 hours to obtain a crystal powder of polymyxin B sulfate (9.23 g), crystal yield: 92.3%.

While the specific embodiments of the present invention has been illustrated and described in detail, it is understood that the present invention is not limited by the specific embodiments described. Various changes, modifications and variations of the present invention are within the scope of the present invention without departing from the spirit and scope of the invention.

## Claims

1. A method for preparing a polymyxin B sulfate crystal, **characterized in that**, said method comprises the following steps of:
1) adsorbing a polymyxin B fermentation liquid with a resin, adding a sulfuric acid aqueous solution and/or an acidic ethanol aqueous solution for desorbing to obtain a desorption liquid;
2) adjusting the pH of the desorption liquid obtained from step 1) to 5.0-7.0, and then concentrating until a solid precipitates to obtain a saturated solution of polymyxin B sulfate; and
3) adding an organic solvent dropwise into the saturated solution of polymyxin B sulfate obtained from step 2), or adding the saturated solution of polymyxin B sulfate obtained from step 2) dropwise into an organic solvent while stirring to precipitate a crystal, filtering, and drying the filter cake to obtain a polymyxin B sulfate crystal.

2. The method according to claim 1, **characterized in that**, in step 1), the concentration of said sulfuric acid aqueous solution is 0.2 mol/L.

3. The method according to claim 1 or 2, **characterized in that**, said method further comprises in step 1), before adsorbing a polymyxin B fermentation liquid with a resin, said polymyxin B fermentation liquid is subjected to a pretreatment which comprises the following steps of:
a) acidifying;
b) adding celite and filtering;
preferably, said adsorbing a polymyxin B fermentation liquid with a resin comprises:
(i) ion exchange treatment;
(ii) macroporous adsorption resin treatment;
more preferably, said pretreatment comprises adjusting the pH of the polymyxin B fermentation liquid to 1.8, then adding celite in step b), filtering after stirring homogeneously, and top washing with water until the potency of the filtrate is less than 0.2 g/L; and still preferably, said adjusting the pH of the polymyxin B fermentation liquid is carried out by adding oxalic acid;
still preferably, said ion exchange treatment comprises adjusting the pH of the filtrate of the polymyxin B fermentation liquid treated by acidifying to 6.7, then adsorbing with a weakly acidic ion exchange resin, and after completely adsorbing, washing with 2-3 volumes of water in terms of column volume until the effluent is colorless, and then desorbing with 0.2 mol/L sulfuric acid aqueous solution; preferably, said weakly acidic ion exchange resin is LXD-135 weakly acidic ion exchange resin; and said adjusting the pH of the filtrate of the polymyxin B fermentation liquid treated by acidifying is carried out with a 2M sodium hydroxide solution;
yet preferably, said macroporous adsorption resin treatment comprises adjusting the pH of the ion exchange treated polymyxin B fermentation liquid to 6.5, and after completely adsorbing by macroporous adsorption resin, top washing with 2 volumes of water in terms of column volume, and then desorbing with an acidic ethanol aqueous solution; preferably, said adjusting the pH of the ion exchange treated polymyxin B fermentation liquid is carried out with a 2M sodium hydroxide solution; and further preferably, said acidic ethanol aqueous solution is a 40 vol% ethanol aqueous solution having a pH of 3;
further preferably, said method further comprises in step 1), concentrating the obtained desorption liquid, decolorizing it by activated carbon, and filtering.

4. The method according to any one of claims 1 to 3, **characterized in that**, in step 2), the pH of the desorption liquid is adjusted to 5.0-7.0 with a 0.5M-10M sodium hydroxide solution.

5. The method according to any one of claims 1 to 4, **characterized in that**, in step 3), said organic solvent is selected from one or more of C₁-C₄ alcohol, C₃-C₄ ketone, ethyl acetate or butyl acetate; preferably, said C₁-C₄ alcohol is selected from one or more of methanol, ethanol, isopropanol, n-propanol or butanol; and still preferably, said C₃-C₄ ketone is selected from one or more of acetone or 2-butanone.

6. The method according to any one of claims 1 to 5, **characterized in that**, in step 3), the process of precipitating a crystal is carried out at a temperature of 0-40 °C;
preferably, the method further comprises in step 3), continuing stirring for 0-8 hours after completion of adding dropwise; and
more preferably, in step 3), said drying is drying under vacuum at a temperature of 40-60 °C for 3-20 hours.

7. A polymyxin B sulfate crystal, **characterized in that**, said polymyxin B sulfate crystal has an X-ray powder diffraction pattern expressed by 2θ degree using Cu-Ka radiation as shown in Figure 3.

8. An antibacterial pharmaceutical composition, **characterized in that**, said pharmaceutical composition comprises the polymyxin B sulfate crystal prepared according to the method according to any one of claims 1 to 6, or the polymyxin B sulfate crystal according to claim 7, and a pharmaceutically acceptable carrier; preferably, said pharmaceutical composition is in the form of tablet, capsule, or granule; more preferably, said tablet is selected from rapid-release tablet, chewable tablet, dispersible tablet, effervescent tablet, sustained release tablet, controlled release tablet or enteric coated tablet, said capsule is selected from hard capsule, soft capsule, sustained release capsule, controlled release capsule or enteric coated capsule, and said granule is selected from suspension granule, effervescent granule, enteric coated granule, sustained release granule or controlled release granule.

9. The pharmaceutical composition according to claim 8, **characterized in that**, said pharmaceutical composition further comprises one or more antibacterial active ingredients other than polymyxin B sulfate.

10. Use of the polymyxin B sulfate crystal prepared according to the method according to any one of claims 1 to 6 or the polymyxin B sulfate crystal according to claim 7 in the preparation of an antibacterial drug; preferably in the preparation of a medicament against Gram-negative bacteria; more preferably, use of the polymyxin B sulfate crystal prepared according to the method according to any one of claims 1 to 6 or the polymyxin B sulfate crystal according to claim 7 in the preparation of a medicament against drug-resistant bacteria, said drug-resistant bacteria are preferably Gram-negative drug-resistant bacteria.
